# EUROPÄISCHE PATENTSCHRIFT

(11) **EP 1 663 269 B1**
(45) Veröffentlichungstag und Bekanntmachung des Hinweises auf die Patenterteilung: **02.01.2008**
(21) Anmeldenummer: 04765026.2
(22) Anmeldetag: 10.09.2004
(51) Int. Cl.: A61K 36/25, A61P 11/00

(54) **VERFAHREN ZUR HERSTELLUNG EINES LAGERSTABILEN EXTRAKTES AUS EFEUBLÄTTERN, SOWIE EIN NACH DIESEM VERFAHREN HERGESTELLTER EXTRAKT**
METHOD FOR THE PRODUCTION OF A STORAGE-RESISTANT IVY LEAF EXTRACT, AND EXTRACT PRODUCED ACCORDING TO SAID METHOD
PROCEDE DE PRODUCTION D'UN EXTRAIT DE FEUILLES DE LIERRE STABLE AU STOCKAGE, ET EXTRAIT OBTENU SELON CE PROCEDE

(30) Priorität: 19.09.2003 DE 10345342
(43) Veröffentlichungstag der Anmeldung: 07.06.2006
(73) Patentinhaber: Engelhard Arzneimittel GmbH & Co. KG, 61138 Niederdorfelden (DE)
(72) Erfinder: RUNKEL, Frank, 35418 Buseck (DE); SCHNEIDER, Wolfgang, 35510 Butzbach (DE); SCHMIDT, Oliver, 35418 Buseck (DE); ENGELHARD, Georg, Maximilian, 61476 Kronberg (DE)
(74) Vertreter: Weller, Wolfgang
(86) Internationale Anmeldenummer: PCT/EP2004/010093
(87) Internationale Veröffentlichungsnummer: WO 2005/037299

(56) Entgegenhaltungen:
- GB-A- 2 051 575
- DATABASE EMBASE [Online] ELSEVIER SCIENCE PUBLISHERS, AMSTERDAM, NL; 1986, WAGNER H ET AL: "Extracts of Hedera helix leaves: HPLC analysis" XP001204524 Database accession no. EMB-1987018570 & DEUTSCHE APOTHEKER ZEITUNG 1986 GERMANY, Bd. 126, Nr. 48, 1986, Seiten 2613-2617,
- DATABASE MEDLINE [Online] US NATIONAL LIBRARY OF MEDICINE (NLM), BETHESDA, MD, US; August 2000 (2000-08), "Green tea." XP002311065 Database accession no. NLM10956382 & ALTERNATIVE MEDICINE REVIEW : A JOURNAL OF CLINICAL THERAPEUTIC. AUG 2000, Bd. 5, Nr. 4, August 2000 (2000-08), Seiten 372-375, ISSN: 1089-5159
- ELIAS R ET AL: "INFLUENCE OF THE DRYING'S PROCESS AND THE ALCOHOLIC DEGREE ON THE EXTRACTION OF HEDERASAPONIN C AND ÄALPHAÜ HEDERIN FROM THE LEACES OF HEDERA HELIX L INFLUENCE DU PROCEDE DE SECHAGE ET DU DEGRE ALCOOLIQUE SUR L'EXTRACTION DE L'HEDERASAPONINE C ET DE L'ALPHA-HEDERINE A PARTIR DES FEUILLES DE HEDERA H" JOURNAL DE PHARMACIE DE BELGIQUE, MASSON, PARIS, FR, Bd. 46, Nr. 3, 1991, Seiten 177-181, XP009033524 ISSN: 0047-2166

## Beschreibung

Die vorliegende Erfindung betrifft ein Verfahren zur Herstellung eines lagerstabilen Extraktes aus getrockneten Efeublättern, unter Verwendung eines Extraktionsmittels, wobei der Extrakt zumindest die Inhaltsstoffe Hederacosid C und α-Hederin aufweist.

Der Begriff "lagerstabil" im Sinne der vorliegenden Erfindung bedeutet, dass ein bestimmtes Hederacosid C/α-Hederin-Verhältnis im Extrakt, sei es durch die natürliche Zusammensetzung des Extraktes bestimmt, sei es durch gezielte Maßnahmen darauf eingestellt, auf lange Zeiträume stabil bleibt.

Extrakte aus Efeublättern werden heutzutage erfolgreich vor allem zur Therapie von Atemwegserkrankungen eingesetzt, da der Extrakt spasmolytische, expektorierende und antiobstruktive Wirkungen zeigt. Diese Wirkungen sind insbesondere auf die therapeutisch wichtigen Inhaltsstoffe der Efeublätterextrakte zurückzuführen, die der Klasse der Triterpensaponine angehören. Hauptsaponin ist dabei das bisdesmosidische Hederacosid C und das daraus durch Esterhydrolyse entstehende α-Hederin. Als weiteres Saponin konnte Hederagenin nachgewiesen werden.

Da Extrakte aus Efeublättern durch verschiedene Verfahren gewonnen werden können, weisen diese Extrakte oftmals unterschiedliche Wirkungsgrade auf. Dies rührt daher, dass der Anteil der Inhaltsstoffe nicht nur von der natürlichen Ausgestaltung der Efeublätter abhängig ist, sondern auch von der jeweiligen Methode der Extraktherstellung abhängt.

In letzter Zeit konnte darüber hinaus durch die Anmelderin nachgewiesen werden, dass insbesondere α-Hederin für die spasmolytische Wirkung von Efeublätter-Extrakten verantwortlich ist, da dieser Wirkstoff an β-adrenerge Rezeptoren bindet, was in der glatten Muskulatur der Bronchialsysteme über weitere Kaskaden zur Erschlaffung führt.

Verfahren zur Herstellung von Trockenextrakten aus Pflanzenmaterialien sind im Gebiet der Pharmazie und insbesondere der Arzneimittelzubereitungen vielfältig beschrieben.

Ein Verfahren zur Herstellung von Trockenextrakten aus Pflanzenmaterialien ist beispielsweise aus der DE 101 12 168 A1 bekannt. Mit dem dort offenbarten Verfahren soll der Gehalt an lipophilen und hydrophilen Substanzen eingestellt werden können. Hierbei wird das Pflanzenmaterial mindestens zwei Extraktionen mit Lösungsmitteln unterschiedlicher Lipophilie unterzogen und die Extrakte daraus separat gewonnen. Die Extrakte werden getrennt voneinander getrocknet und im gewünschten Verhältnis gemischt. Auf diese Weise kann der Gehalt an lipophilen und hydrophilen Substanzen eingestellt werden. Das Verfahren soll auch zur Gewinnung von Trockenextrakten aus Efeu (Hedera helix) geeignet sein.

Nachteilig an diesem Verfahren ist jedoch der Umstand, dass zwei getrennte Extraktionen durchgeführt werden müssen, wodurch das Verfahren insgesamt sehr aufwändig wird.

Ferner ist aus der DE 30 25 223 A1 eine pharmazeutische Zubereitung auf der Grundlage von Efeuextrakten und einem Verfahren zu deren Herstellung bekannt, bei welchem unter Verwendung von Aceton und Methanol ein Efeuextrakt mit einem Hederasaponin C-Gehalt von 60 bzw. 90 % gewonnen wird.

Der aus diesem Verfahren gewonnene Extrakt ist jedoch in seinem Hederacosid C-Gehalt nicht stabil, da erfahrungsgemäß Hederacosid C im Extrakt zu α-Hederin hydrolysiert wird.

Oftmals besteht jedoch die Notwendigkeit, den Gehalt an den Inhaltsstoffen Hederacosid C und α-Hederin in Efeuextrakten stabil aufrecht und damit lagerbar zu erhalten. Denn nur mit stabil vorliegenden Mengenverhältnissen lassen sich bspw.

Mischextrakte mit genau eingestellten Konzentrationsverhältnissen einstellen. Derartige genau eingestellte Konzentrationsverhältnisse erleichtern die Herstellung von Arzneimitteln mit exakt reproduzierbaren Wirkstoffanteilen und eröffnen die Möglichkeit, auf die Bioverfügbarkeit Einfluss zu nehmen.

Aufgabe der vorliegenden Erfindung ist es daher, ein Verfahren bereitzustellen, mit welchem mit wenigen Schritten und ohne großen Aufwand ein Efeu-Extrakt, insbesondere ein Trockenextrakt, gewonnen werden kann, in welchem der Gehalt an den Inhaltsstoffen Hederacosid C und α-Hederin lagerstabil vorliegt.

Gemäß dem eingangs genannten Verfahren wird die der Erfindung zu Grunde liegende Aufgabe dadurch gelöst, dass das Verfahren die folgenden Schritte aufweist:
a) Zerkleinern einer Menge an getrockneten Efeublättern,
b) Bedampfen der zerkleinerten Menge an getrockneten Efeublättern mit heißem Wasserdampf,
c) Extrahieren durch Zugabe des Extraktionsmittels, und
d) ggf. Trocknen des Extraktes.

Mit dem erfindungsgemäßen Verfahren kann nunmehr ein Extrakt aus getrockneten Efeublättern gewonnen werden, welcher die Inhaltsstoffe Hederacosid und α-Hederin in einer lagerstabilen Konzentration aufweist. Die unerwünschte Umwandlung von Hederacosid C/α-Hederin und somit die Änderung des Hederacosid C/α-Hederin-Verhältnisses im Extrakt ist ausgeschlossen.

Insbesondere kann mit diesem Verfahren erreicht werden, dass ein Extrakt hergestellt wird, der maximal die in der Ausgangsdroge bereits vorliegende Menge an α-Hederin aufweist. Anders ausgedrückt wird ein Extrakt gewonnen, der einen hohen Hederacosid C-Gehalt besitzt, der sich über längere Zeiträume nicht verändert. Solche Extrakte sind bspw. dann von großem Interesse, wenn diese Extrakte als Ausgangsmaterial für Mischextrakte dienen sollen, die einen konkret bestimmbaren und exakt festgestellten Gehalt an den Wirkstoffen Hederacosid C und α-Hederin aufweisen.

Insbesondere durch den Verfahrensschritt des Bedampfens wird ferner die Extraktionsausbeute an Hederacosid C erhöht, da durch die Bedampfung die Zellen in den Efeublättern aufgeschlossen werden und dadurch das die Umwandlung von Hederacosid C in α-Hederin katalysierende Enzym zum großen Teil inaktiviert wird.

Je nach Einsatz der Ausgangsdroge, bzw. je nach Gehalt der in der Ausgangsdroge vorliegenden Wirkstoffe, können demnach durch das erfindungsgemäße Verfahren Extrakte mit hohem Hederacosid C-Gehalt und niedrigem α-Hederin-Gehalt gewonnen werden. Der Prozentgehalt an α-Hederin im nach dem erfindungsgemäßen Verfahren gewonnenen Extrakt übersteigt nicht den Prozentgehalt innerhalb der eingesetzten Blätter. Demnach weist bspw. ein erhaltener Extrakt, für dessen Gewinnung Blattchargen mit einem Gehalt von unter 0,5 % α-Hederin eingesetzt wurden, ein Höchstgehalt von 0,5 % α-Hederin auf.

Ein derartiger Extrakt kann dann vorteilhaft bspw. für die gezielte Herstellung von Mischungen an Extrakten eingesetzt werden, die jeweils genau bestimmbare Mengen an den jeweiligen Wirkstoffen - Hederacosid C und α-Hederin - aufweisen, so dass Endextrakte gewonnen werden, die einen konkret spezifizierten Gehalt an beiden Wirkstoffen enthalten. Dieser Bedampfungsschritt eröffnet auch die Möglichkeit, zunächst eine gewisse Umwandlung von Hederacosid C in α-Hederin zuzulassen, dann aber abzubrechen.

Bei dem erfindungsgemäßen Verfahren werden die Efeublätter als getrocknete Droge eingesetzt.

Getrocknete Drogen haben in der Arzneimittelherstellung den Vorteil, dass sie bezüglich der Haltbarkeit u.U. besser zu handhaben sind als frische Drogen.

Getrocknete Arzneipflanzen und Arzneipflanzenteile werden im Gebiet der Arzneimitteltechnik definitionsgemäß als "Drogen" bezeichnet. Die Verwendung von solchen als "Drogen" vorliegenden Arzneipflanzen erfolgt dabei entweder in unveränderter oder in zerkleinerter Form.

Weiterhin ist bevorzugt, wenn in Schritt a) eine Zerkleinerung der getrockneten Efeublätter auf ≤ 5 x 5 mm erfolgt.

Ferner ist bevorzugt, wenn das Bedampfen über einen Zeitraum von 1 bis 30 Sekunden, insbesondere von 1 bis 10 Sekunden und bevorzugt mit 5 Sekunden durchgeführt wird.

Dieser Zeitraum erwies sich als für das Extraktionsverfahren als effektiv, da zum einen die Hydrolyse von Hederacosid C zu α-Hederin unterbunden und gleichzeitig eine zügige Durchführung des Verfahrens gewährleistet wird.

Hierbei ist bevorzugt, wenn das Bedampfen mit Wasserdampf bei einer Temperatur von ca. 90°C (ggf. bei Unterdruck) bis 140°C erfolgt, insbesondere bei einer Temperatur von ca. 100 bis 120°C, und bevorzugt bei 110°C.

Bei dem erfindungsgemäßen Verfahren ist ferner bevorzugt, wenn das Extraktionsmittel ein Gemisch aus Alkohol- und Wasseranteilen ist.

Insbesondere ist bevorzugt, wenn als Alkohol Ethanol 30 % eingesetzt wird.

Ethanol ist ein bewährtes pharmazeutisches Lösungsmittel und wird im Gebiet der Arzneimitteltechnik als Extraktionsmittel vielfältig eingesetzt. Selbstverständlich können aber auch andere Alkohole, bspw. Propanol, Isopropanol, etc. eingesetzt werden, also alle Alkohole, die als Wasser-/Alkoholgemische bei der Extraktion im Sinne der Arzneimittelzubereitung einsetzbar sind.

Weiterhin ist bevorzugt, wenn das Extrahieren in Schritt c) durch Vorquellen und Perkolation durchgeführt wird, und insbesondere, wenn für das Vorquellen ein Zeitraum von ca. 1 Stunde bis ca. 30 Stunden, vorzugsweise ein Zeitraum von 6 Stunden, vorgesehen ist.

Vorquellen und Perkolation sind im Stand der Technik und insbesondere auf dem Gebiet der Arzneimittelzubereitungen aus Pflanzen gängige Methoden. Mit den Schritten des Vorquellens und der Perkolation kann eine erschöpfende Extraktion der Efeublätter erzielt werden.

Dabei ist bevorzugt, wenn eine Verfahrenstemperatur von 10 bis 40 °C, insbesondere von 30 °C, eingehalten wird.

In einer Weiterführung des erfindungsgemäßen Verfahrens ist bevorzugt, wenn das Trocknen in Schritt d) durch Dünnfilmvaporisation und anschließender Sprühtrocknung durchgeführt wird.

Beide Verfahren sind herkömmliche Trocknungsverfahren, wobei sich die Dünnfilmvaporisation bei der Zubereitung von Arzneimitteln als schonendes Verdampfungsverfahren erwiesen hat, ebenso wie die Sprühtrocknung, mit welcher sich erfahrungsgemäß flüssige Zubereitungen zu pulverförmigen Endprodukten trocknen lassen, die sich insbesondere dadurch auszeichnen, dass sie sich beispielsweise mit Wasser wieder leicht zu gebrauchsfertigen Zubereitungen anrühren lassen. Die Dünnfilmvaporisation wird dabei bspw. bei ca. 50 bis 60°C und bei 130 bis 180 mbar durchgeführt, und die Sprühtrocknung bei 45 bis 60°C.

Ferner ist bevorzugt, wenn das erfindungsgemäße Verfahren zur Herstellung eines Extraktes aus getrockneten Efeublättern mit erhöhtem Hederacosid C-Gehalt eingesetzt wird.

Mit dem erfindungsgemäßen Verfahren ist es wie oben dargestellt möglich, den Anteil an Hederacosid C gezielt zu erhöhen, wobei gleichzeitig der Gehalt an α-Hederin im Extrakt maximal dem Gehalt an α-Hederin in der Ausgangsdroge entspricht.

In einer Ausgestaltung ist vorgesehen, vor dem Schritt b), also der Bedampfung, eine gezielte Umwandlung von zumindest eines Teils des Hederacosids C in α-Hederin zu bewirken.

Dies eröffnet die Möglichkeit, Extrakte mit einem bestimmten Hederacosid C/α-Hederin-Verhältnis gezielt herzustellen (quasi zu "designen") und erst nach Erreichen eines bestimmten Hederacosid C/α-Hederin-Verhältnis den Bedampfungsschritt durchzuführen, der dann eine weitere Umwandlung von Hederacosid C in α-Hederin unterbindet.

Dies beruht auf der Erkenntnis der Anmelderin, dass durch einen Fermentierungsschritt, der durch Wasser katalysiert wird, eine Umwandlung von Hederacosid C zu α-Hederin bewirkt werden kann.

Die Erfindung betrifft ferner einen lagerstabilen Extrakt aus getrockneten Efeublättern, der nach dem erfindungsgemäßen Verfahren hergestellt ist.

Extrakte mit einem hohen Gehalt an Hederacosid sind bspw. dann von Vorteil, wenn Mischungen aus Extrakten hergestellt werden sollen, die einen bestimmbaren Gehalt an Hederacosid aufweisen. Auch wenn, wie weiter oben ausgeführt, α-Hederin der wirksame Bestandteil der Efeublätter im Sinne von bronchospasmolytisch darstellt, sind auch Extrakte mit einem hohen Gehalt an Hederacosid C vorteilhaft, da diese - wie oben erwähnt - für gezielte Mischungen eingesetzt werden können.

Die Erfindung betrifft ferner die Verwendung des erfindungsgemäß hergestellten Extraktes, insbesondere des Trockenextraktes, zur Herstellung eines Arzneimittels, und insbesondere zur Herstellung eines Arzneimittels zur Behandlung von Atemwegserkrankungen.

Das erfindungsgemäße Arzneimittel kann danach zur Behandlung von Atemwegserkrankungen wie bspw. infektiös-entzündliche Atemwegserkrankungen eingesetzt werden, wie bspw. Pneumonie, Tracheitis, Bronchitis etc., aber auch bei obstruktiven und restriktiven Lungenerkrankungen wie chronische Bronchitis, Asthma bronchiale, Bronchiektasen usw., also bei Atemwegserkrankungen, bei welchen eine Relaxierung der schwachen Muskulatur erwünscht ist.

Weiterhin betrifft die Erfindung ein Arzneimittel, welches einen Extrakt aufweist, der nach dem erfindungsgemäßen Verfahren hergestellt ist.

Dabei kann das Arzneimittel in Form von Kapseln, Tabletten, Dragees, Zäpfchen, Granulat, Pulver, Lösungen, Cremes, Emulsionen, Aerosolen, Salben und Ölen vorliegen. Orale Verabreichungsformen sind dabei insbesondere bevorzugt. Das Arzneimittel kann dabei Hilfsstoffe aufweisen, die herkömmlich in der Arzneimittelherstellung verwendet werden. Eine Reihe von geeigneten Substanzen findet sich bspw. in A. Kibbe, Handbook of Pharmaceutical Excipients, 3. Ed., 2000, American Pharmaceutical Association and pharmaceutical Press.

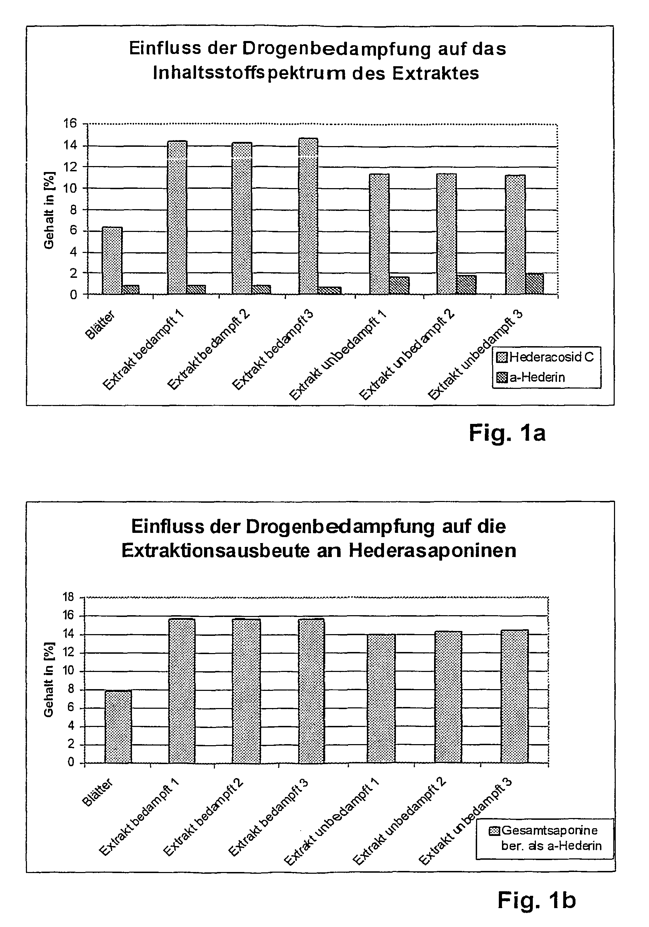

Weitere Vorteile ergeben sich aus dem Beispiel und der beigefügten Figuren. Es zeigen
- Fig. 1a: den Einfluss der Drogenbedampfung auf das Inhaltsstoffspektrum des Extraktes in grafischer Darstellung
- Fig. 1b: den Einfluss der Drogenbedampfung auf die Extraktionsausbeute an Hederasaponinen in grafischer Darstellung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Beispiel:

### Herstellung von Efeublättertrockenextrakten mit erhöhtem Hederacosid C-Gehalt

Grundlage für die Steuerung des Verhältnisses von Hederacosid C zu α-Hederin ist es, die Abspaltung des C28-ständigen Zuckers des Hederacosid C während des Extraktionsverfahrens gezielt zu unterbinden. Bei der Auswahl der Drogencharge sollte darauf geachtet werden, dass Blattchargen mit niedrigem α-Hederin-Gehalt eingesetzt werden. Eine Spezifizierung auf unter 0,5 % α-Hederin in Bezug auf die getrocknete Droge ist empfehlenswert.

Eine homogene Probe Efeublätter wurde bezüglich des Gehalts der beiden Saponine wie folgt analysiert:

| | |
|---|---|
| **Hederacosid C:** | **6,37 %** |
| **α-Hederin:** | **0,85 %** |

Ausgehend von der Droge wurden zweimal je drei Extraktionen nach folgenden Extraktionsvorschriften durchgeführt:

### Extraktionsvorschrift 1:

3 g der getrockneten und auf ca. 3 x 3 mm zerkleinerten Droge wurden mit heißem Wasserdampf (ca. 100°C) für einige Sekunden bedampft. Die so behandelte Droge wurde mit 18 g des Extraktionsmittels (Ethanol 30 % (m/m))ca. 6 Stunden vorgequollen. Nach Ablassen der Miscella wurde die verbleibende Droge mit weiteren 18 g des Extraktionsmittels perkoliert. Diese Miscella wurde im Vakuumtrockenschrank getrocknet.

Die Extraktionstemperatur liegt bevorzugt zwischen ca. 20 und ca. 40°C, insbesondere bei ca. 30 °C. Das Verhältnis von Droge zum Extraktionsmittel liegt dabei bspw. bei 1:12.

Alternativ kann die Trocknung bspw. durch Dünnfilmvaporisation bei bspw. 55 °C und 150 mbar und anschließender Sprühtrocknung bei 45 - 60 °C erfolgen.

### Extraktionsvorschrift 2:

Die Extraktionsvorschrift entspricht der Extraktionsvorschrift 1 mit Ausnahme des Verfahrensschrittes der Bedampfung.

Nach Analyse der erhaltenen Extrakte konnten die in der folgenden Tabelle 1 aufgeführten Ergebnisse dokumentiert werden:

**Tabelle 1: Gehalt an Hederacosid C und α-Hederin in den Extrakten**

| Probe | Gehalt Hederacosid C | Gehalt α-Hederin | Gehalt Hederasaponine, berechnet als Hederacosid C |
|---|---|---|---|
| | [%] | [%] | [%] |
| Ausgangsdroge | 6,37 | 0,85 | 7,75 |
| Extrakt 1 bedampft | 14,33 | 0,85 | 15,71 |
| Extrakt 2 bedampft | 14,26 | 0,85 | 15,64 |
| Extrakt 3 bedampft | 14,57 | 0,71 | 15,72 |
| Extrakt 1 unbedampft | 11,39 | 1,61 | 14,00 |
| Extrakt 2 unbedampft | 11,35 | 1,82 | 14,30 |
| Extrakt 3 unbedampft | 11,30 | 1,92 | 14,42 |

In den Fig. 1a und 1b sind die Ergebnisse durch die beiden Grafiken verdeutlicht. Die Figuren zeigen deutlich, dass in dem nach der Extraktionsvorschrift 1 (mit Bedampfungsschritt) hergestellten Extrakt ein höherer Hederacosid C-Gehalt und ein niedrigerer α-Hederin-Gehalt vorliegt als bei dem mit einem herkömmlichen Verfahren hergestellten Extrakt (ohne Bedampfungsschritt) (siehe Fig. 1a).

Gleichzeitig konnte die Extraktionsausbeute mit der Extraktionsvorschrift 1 (mit Bedampfungsschritt) im Vergleich zur Extraktionsvorschrift 2 (ohne Bedampfungsschritt) erhöht werden (siehe Fig. 1b).

Der nach dieser Vorschrift hergestellte Blättertrockenextrakt wies demnach die maximal in den eingesetzten Blättern enthaltene Menge an α-Hederin. Insgesamt wurden somit Extrakte mit hohem Hederacosid C-Gehalt und sehr niedrigem α-Hederin-Gehalt erhalten. Bei Verwendung von Blattchargen mit einem Gehalt von unter 0,5 % α-Hederin kann auch für den erhaltenen Extrakt ein Höchstgehalt von 0,5 % α-Hederin angenommen werden.

Durch gezieltes Mischen verschiedener mit Hederacosid angereicherter Extrakte kann somit ein Extrakt erhalten werden, der sich durch einen spezifizierten Gehalt an Hederacosid C und einen Maximalgehalt von 0,5 % α-Hederin kennzeichnet.

Diese Trockenextrakte wurden gelagert und nach 3, 6 und 9 Monaten wurde das Hederacosid C/α-Hederin-Verhältnis erneut bestimmt. Dabei wurde keine signifikante Änderung des Verhältnisses festgestellt.

Diese gelagerten Extrakte können bei späteren Formulierungen zu Arzneimitteln problemlos eingesetzt werden. Eine unerwünschte Umwandlung von Hederacosid C in α-Hederin kann ausgeschlossen werden, so dass gezielt und reproduzierbar Arzneimittel mit bestimmtem Hederacosid C/α-Hederin-Verhältnis hergestellt werden können.

## Patentansprüche

1. Verfahren zur Herstellung eines lagerstabilen Extraktes aus Efeublättern unter Verwendung eines Extraktionsmittels, wobei der Extrakt zumindest die Inhaltsstoffe Hederacosid C und α-Hederin aufweist, **dadurch gekennzeichnet, dass** es die folgende Schritte aufweist:
a) zerkleinern einer Menge an getrockneten Efeublättern;
b) Bedampfen der zerkleinerten Menge an getrockneten Efeublättern mit heißem Wasserdampf;
c) Extrahieren durch Zugabe des Extraktionsmittels, und
d) ggf. Trocknen des Extraktes.

2. Verfahren nach Anspruch 1, **dadurch gekennzeichnet, dass** in Schritt a) eine Zerkleinerung der Efeublätter auf ≤ 5 x 5 mm erfolgt.

3. verfahren nach einem der Ansprüche 1 bis 2, **dadurch gekennzeichnet, dass** das Bedampfen über einen Zeitraum von 1 bis 30 Sekunden durchgeführt wird.

4. Verfahren nach Anspruch 3, **dadurch gekennzeichnet, dass** das Bedampfen für einen Zeitraum von 1 bis 10 Sekunden, insbesondere für 5 Sekunden, durchgeführt wird.

5. Verfahren nach einem der Ansprüche 1 bis 4, **dadurch gekennzeichnet, dass** das Bedampfen mit Wasserdampf bei einer Temperatur von ca. 90°C bis 140°C erfolgt.

6. Verfahren nach Anspruch 5, **dadurch gekennzeichnet, dass** das Bedampfen mit Wasserdampf bei einer Temperatur von ca. 100 bis ca. 120°C, insbesondere von 110 °C, erfolgt.

7. Verfahren nach einem der Ansprüche 1 bis 6, **dadurch gekennzeichnet, dass** als Extraktionsmittel ein Alkohol/Wasser-Gemisch eingesetzt wird.

8. Verfahren nach Anspruch 7, **dadurch gekennzeichnet, dass** als Extraktionsmittel Ethanol 30 % eingesetzt wird.

9. verfahren nach einem der Ansprüche 1 bis 8, **dadurch gekennzeichnet, dass** das Extrahieren durch vorquellen und Perkolation durchgeführt wird.

10. Verfahren nach Anspruch 9, **dadurch gekennzeichnet, dass** das Vorquellen für einen Zeitraum von ca. 1 bis ca. 30 Stunden durchgeführt wird.

11. Verfahren nach Anspruch 10, **dadurch gekennzeichnet, dass** das vorquellen für einen Zeitraum von ca. 6 h durchgeführt wird.

12. verfahren nach einem der Ansprüche 1 bis 11, **dadurch gekennzeichnet, dass** das Trocknen durch Dünnfilmvaporisation und anschließender Sprühtrocknung durchgeführt wird.

13. verfahren nach einem der Ansprüche 1 bis 12, zur Herstellung eines Extraktes aus Efeublättern mit einem α-Hederin-Gehalt von ≤ 0,85 %.

14. Verfahren nach einem der Ansprüche 1 bis 12, **dadurch gekennzeichnet, dass** vor dem Schritt b) eine gezielte Umwandlung eines Teils des Hederacosids C in α-Hederin bewirkt wird.

15. Extrakt aus Efeublättern, **dadurch gekennzeichnet, dass** er gemäß dem Verfahren nach einem der Ansprüche 1 bis 14 hergestellt ist.

16. Verwendung eines Extraktes nach Anspruch 15 zur Herstellung eines Arzneimittels.

17. Verwendung nach Anspruch 16, **dadurch gekennzeichnet, dass** das Arzneimittel zur Behandlung von Atemwegserkrankungen eingesetzt wird.

18. Arzneimittel, welches einen Extrakt nach Anspruch 15 aufweist.

## Claims

1. Method for preparing a storage-stable ivy leaves extract using an extracting agent, with the extract comprising at least the constituents hederacoside C and α-hederin, **characterized in that** it comprises the following steps:
a) comminuting a quantity of dried ivy leaves;
b) steaming the comminuted quantity of dried ivy leaves with hot water vapor;
c) extracting by adding the extracting agent, and
d) where appropriate, drying the extract.

2. Method of claim 1, **characterized in that** the ivy leaves are comminuted down to ≤ 5 x 5 mm in step a).

3. Method of claim 1 or 2, **characterized in that** the steaming is carried out over a period of from 1 to 30 seconds.

4. Method of claim 3, **characterized in that** the steaming is carried out for a period of from 1 to 10 seconds, in particular for 5 seconds.

5. Method of anyone of claims 1 through 4, **characterized in that** the steaming with water vapor is carried out at a temperature of from approx. 90°C to 140°C.

6. Method of claim 5, **characterized in that** the steaming with water vapor is carried out at a temperature of from approx. 100°C to approx. 120°C, in particular of 110°C.

7. Method of anyone of claims 1 through 6, **characterized in that** the extracting agent employed is an alcohol/water mixture.

8. Method of claim 7, **characterized in that** the extracting agent employed is 30% ethanol.

9. Method of anyone of claims 1 through 8, **characterized in that** the extracting is carried out by means of preswelling and percolation.

10. Method of claim 9, **characterized in that** the preswelling is carried out for a period of from approx. 1 to approx. 30 hours.

11. Method of claim 10, **characterized in that** the preswelling is carried out for a period of approx. 6 h.

12. Method of anyone of claims 1 through 11, **characterized in that** the drying is carried out by thin film vaporization and subsequent spray drying.

13. Method of anyone of claims 1 through 12 for preparing an ivy leaves extract having a content of α-hederin ≤ 0.85 %.

14. Method of anyone of claims 1 through 12, **characterized in that** at least a part of the hederacoside C is selectively converted into α-hederin prior to step b).

15. Ivy leaves extract, **characterized in that** it is prepared using the method as claimed in anyone of claims 1 through 14.

16. Use of an extract of claim 15 for producing a pharmaceutical.

17. Use of claim 16, **characterized in that** the pharmaceutical is used for treating respiratory tract diseases.

18. Pharmaceutical which comprises an extract as claimed in claim 15.

## Revendications

1. Procédé de production d'un extrait de feuilles de lierre stable au stockage en utilisant un agent d'extraction, dans lequel l'extrait présente au moins les ingrédients hédéracoside C et α-hédérine, **caractérisé en ce qu'**il comporte les étapes suivantes :
a) broyer en petits morceaux une quantité de feuilles de lierre séchées ;
b) soumettre la quantité broyée de feuilles de lierre séchées à de la vapeur d'eau chaude ;
c) réaliser l'extraction par ajout de l'agent d'extraction ; et
d) écher éventuellement l'extrait.

2. Procédé selon la revendication 1, **caractérisé en ce que** dans l'étape a) se produit un broyage des feuilles de lierre à une taille ≤ 5 x 5 mm.

3. Procédé selon l'une des revendications 1 à 2, **caractérisé en ce que** l'exposition à la vapeur est conduite sur une durée comprise entre 1 et 30 secondes.

4. Procédé selon la revendication 3, **caractérisé en ce que** l'exposition à la vapeur est conduite sur une durée comprise entre 1 et 10 secondes, et en particulier de 5 secondes.

5. Procédé selon l'une des revendications 1 à 4, **caractérisé en ce que** l'exposition à la vapeur est effectuée avec de la vapeur d'eau à une température comprise entre environ 90 °C et 140 °C.

6. Procédé selon la revendication 5, **caractérisé en ce que** l'exposition à la vapeur est effectuée avec de la vapeur d'eau à une température comprise entre environ 100 et environ 120 °C, en particulier à 110 °C.

7. Procédé selon l'une des revendications 1 à 6, **caractérisé en ce que** l'on utilise en tant qu'agent d'extraction un mélange alcool/eau.

8. Procédé selon la revendication 7, **caractérisé en ce que** l'on utilise en tant qu'agent d'extraction de l'éthanol à 30 %.

9. Procédé selon l'une des revendications 1 à 8, **caractérisé en ce que** l'extraction est réalisée par pré-gonflement et percolation.

10. Procédé selon la revendication 9, **caractérisé en ce que** le pré-gonflement est effectué pendant une durée comprise entre environ 1 et environ 30 heures.

11. Procédé selon la revendication 10, **caractérisé en ce que** le pré-gonflement est effectué pendant une durée d'environ 6 heures.

12. Procédé selon l'une des revendications 1 à 11, **caractérisé en ce que** le séchage est réalisé par évaporation sur couche mince puis séchage par pulvérisation.

13. Procédé selon l'une des revendications 1 à 12 pour produire un extrait de feuilles de lierre présentant une teneur en α-hédérine ≤ 0,85 %.

14. Procédé selon l'une des revendications 1 à 12, **caractérisé en ce qu'**avant l'étape b), on provoque une conversion ciblée d'une partie de l'hédéracoside C en α-hédérine.

15. Extrait de feuilles de lierre, **caractérisé en ce qu'**il est produit selon le procédé selon l'une des revendications 1 à 14.

16. Utilisation d'un extrait selon la revendication 15 pour produire un médicament.

17. Utilisation selon la revendication 16, **caractérisée en ce que** le médicament est utilisé pour le traitement de maladies des voies respiratoires.

18. Médicament qui contient un extrait selon la revendication 15.
